(19) **Europäisches Patentamt**

**European Patent Office**

**Office européen des brevets**

(11) **EP 1 702 562 A1**

(12) **EUROPEAN PATENT APPLICATION**

(43) Date of publication:
**20.09.2006 Bulletin 2006/38**

(51) Int Cl.:
*A61B 5/022* (2006.01)

(21) Application number: **06005217.2**

(22) Date of filing: **14.03.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **16.03.2005 JP 2005075535**

(71) Applicant: **Omron Healthcare Co., Ltd.**
**Kyoto 615-0084 (JP)**

(72) Inventors:
• **Sano, Yoshihiko**
**Omron Healthcare Co., Ltd.**
**Kyoto-shi**
**Kyoto 615-0084 (JP)**
• **Karo, Hiromichi**
**Omron Healthcare Co., Ltd.**
**Kyoto-shi**
**Kyoto 615-0084 (JP)**
• **Kishimoto, Hiroshi**
**Omron Healthcare Co., Ltd.**
**Kyoto-shi**
**Kyoto 615-0084 (JP)**
• **Tsurumi, Yoshinori**
**Omron Healthcare Co., Ltd.**
**Kyoto-shi**
**Kyoto 615-0084 (JP)**

(74) Representative: **Kilian, Helmut**
**Wilhelms, Kilian & Partner**
**Patentanwälte**
**Eduard-Schmid-Strasse 2**
**81541 München (DE)**

(54) **Blood pressure monitor cuff and blood pressure monitor**

(57)  A curled elastic member (160) has a flexion portion (161) allowing for local flexion. The curled elastic member (160) is provided with an abutment piece (165) positioned on the outer side of the flexion portion (161) and brought into abutment with the outer surface in proximity to the flexion portion (161) when the curled elastic member (160) is in the shape suitable for blood pressure measurement. The flexion portion (161) is provided with a groove (162) on the inner circumferential side thereof with a groove bottom having the shape of an arc in cross section. A blood pressure monitor cuff and a blood pressure monitor are provided with the curled elastic member formed to be foldable without losing the essentially required characteristics.

FIG.8

Printed by Jouve, 75001 PARIS (FR)

**Description**

BACKGROUND OF THE INVENTION

Field of the Invention

The present invention relates to a blood pressure monitor cuff with an air bag for squeezing a living body to interrupt the blood flow through an artery and a blood pressure monitor including the same.

Description of the Background Art

Usually, in order to measure blood pressure, a cuff containing a fluid bag for squeezing an artery in a living body is first wrapped around the surface of the living body. Pressure is then applied to the wrapped fluid bag and reduced as appropriate, so that an artery pulse pressure and wave in the artery is detected. The blood pressure is thus measured.

A cuff refers to a hollow strap-like structure having a fluid bag that can be wrapped around a part of a living body. The cuff includes a fluid bag, wrapping means for wrapping the fluid bag around a living body, and a curled elastic member for keeping the shape of the cuff. A cuff wrapped around and fitted on the wrist or upper arm of a human body is sometimes called an armband or a manchette.

The curled elastic member used for such a cuff is usually integrally formed of a synthetic resin having elasticity. Now, blood pressure monitors with good portability have been developed in order to measure blood pressure anywhere at any time. For this purpose, a curled elastic member that also allows for compact storage is desirable. In order to solve the aforementioned problems, Japanese Patent Laying-Open No. 06-105813 proposes a foldable cuff.

In the cuff described in Japanese Patent Laying-Open No. 06-105813, a concave groove is provided on the outer face of the curled elastic member, and a hinge portion is formed by providing the concave groove. The hinge portion allows the curled elastic member to be freely folded inwardly, and mutual abutment between the side walls of the concave groove prevents the curled elastic member from unfolding outwardly.

In the curled elastic member described in Japanese Patent Laying-Open No. 06-105813, the mutual abutment between the inner surfaces of the concave groove prevents unfolding outwardly further than a prescribed angle. In order to realize this function, the curled elastic member must be considerably thick.

However, the thickness of the curled elastic member suitable for obtaining an optimum holding force when fitted on a human body does not always agree with the thickness of the curled elastic member suitable for forming the aforementioned hinge portion. Therefore, when the thickness of the curled elastic member is determined so as to attain the function as a hinge portion, the optimum holding force cannot be obtained, or the position at which the hinge portion is provided is restricted.

SUMMARY OF THE INVENTION

It is an object of the present invention to provide a blood pressure monitor cuff and a blood pressure monitor with a curled elastic member formed to be foldable without losing the characteristics essentially required of the curled elastic member.

In accordance with the present invention, a blood pressure monitor cuff includes a curled elastic member having a flexion portion allowing for local flexion. The curled elastic member is provided with an abutment piece positioned on the outer side of the flexion portion and brought in abutment with the outer surface in proximity to the flexion portion when the curled elastic member is in the shape suitable for blood pressure measurement.

With this configuration, the abutment piece is provided on the outer side of the flexion portion and is brought into abutment with the outer surface in proximity to the flexion portion, so that the flexion portion is prevented from unfolding outwardly because of the abutment with the abutment piece. Therefore, the shape suitable for blood pressure measurement is kept when the flexion portion is opened. In addition, provision of the abutment piece prevents outward unfolding, so that this function can be realized irrespective of the thickness of the curled elastic member at the flexion portion. Accordingly, the optimum thickness of the curled elastic member can be adopted to obtain the functional characteristics essentially required of the curled elastic member.

In the blood pressure monitor cuff as described above, the flexion portion may have a groove provided on the inner circumference thereof and extending in the direction orthogonal to the circumferential direction of the curled elastic member. Furthermore, the groove bottom of the groove may be formed of a curved surface. Because of this configuration, stress concentration in the flexion portion can be reduced when the curled elastic member is folded.

In the blood pressure monitor cuff as described above, the flexion portion may be formed such that an area per unit length in the circumferential direction of the curled elastic member is smaller than that of any other portion of the curled elastic member. The area of the flexion portion is reduced by providing an opening or cut at the flexion portion, so that the flexural rigidity of the flexion portion is reduced and the flexion portion can be flexed easily.

A blood pressure monitor may be formed of the blood pressure monitor cuff as described above, a fluid bag inflated and deflated as a fluid comes in and out, an inflation/deflation unit inflating and deflating the fluid bag, a pressure detection unit detecting a pressure in the fluid bag, and a blood pressure value calculation unit calculating a blood pressure value based on information of the pressure detected by the pressure detection unit.

The present invention provides a blood pressure monitor cuff and a blood pressure monitor with a curled elastic member formed to be foldable without losing the characteristics essentially required of the curled elastic member.

The foregoing and other objects, features, aspects and advantages of the present invention will become more apparent from the following detailed description of the present invention when taken in conjunction with the accompanying drawings.

BRIEF DESCRIPTION OF THE DRAWINGS

Fig. 1 is an external perspective view of a blood pressure monitor in a first embodiment in accordance with the present invention.

Fig. 2 is a cross sectional view showing an interior structure of a blood pressure monitor cuff in the first embodiment in accordance with the present invention.

Fig. 3 is a block diagram showing a configuration of the blood pressure monitor in the first embodiment in accordance with the present invention.

Fig. 4 is a flowchart showing a flow of a blood pressure measurement process in the first embodiment in accordance with the present invention.

Fig. 5 is a front view showing a structure of a curled elastic member in the first embodiment in accordance with the present invention.

Fig. 6 is a side view showing the structure of the curled elastic member in the first embodiment in accordance with the present invention.

Fig. 7 is a front view showing a configuration of a flexion portion in the first embodiment in accordance with the present invention.

Fig. 8 is a cross sectional view showing that the curled elastic member is folded in the first embodiment in accordance with the present invention.

Fig. 9 is a perspective view showing a structure of a flexion portion of a curled elastic member in a second embodiment in accordance with the present invention.

Fig. 10 is a front view showing the structure of the flexion portion of the curled elastic member in the second embodiment in accordance with the present invention.

DESCRIPTION OF THE PREFERRED EMBODIMENT

In the following, a structure of a blood pressure monitor cuff and a blood pressure monitor in each embodiment in accordance with the present invention will be described with reference to the figures. It is noted that in the embodiments the same or corresponding parts will be denoted with the same reference characters and the description thereof will not be repeated.

(First Embodiment)

In the following embodiment, a wrist blood pressure monitor will be described by way of illustration. However, the blood pressure monitor cuff and the blood pressure monitor in accordance with the present invention are not limited to a wrist blood pressure monitor and is applicable to any other blood pressure monitor such as an upper arm blood pressure monitor.

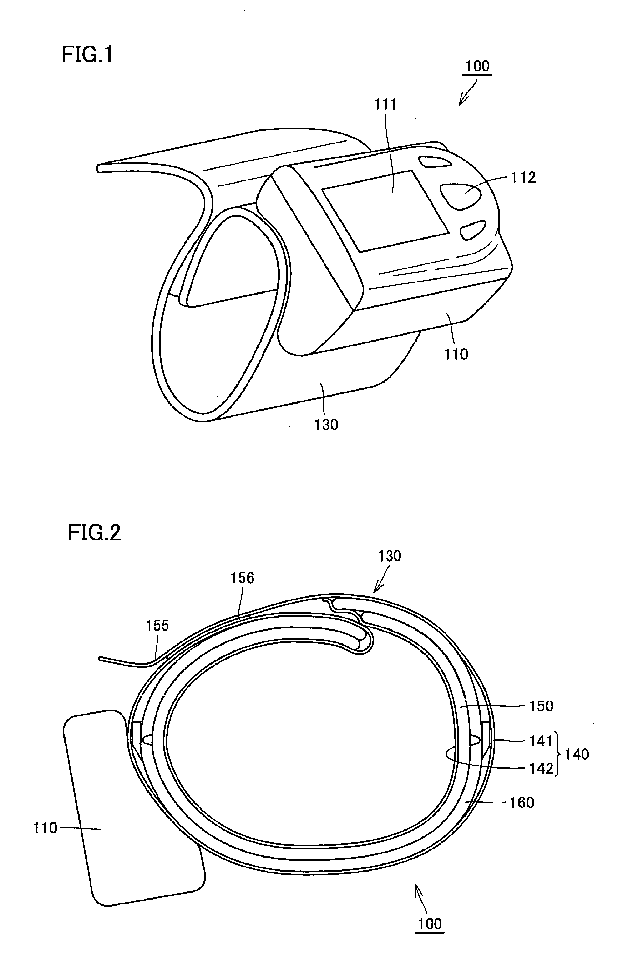

Fig. 1 is an external perspective view of a blood pressure monitor in an embodiment of the present invention. As shown in Fig. 1, a blood pressure monitor 100 in the embodiment of the present invention includes an apparatus body 110 and a cuff 130. A display unit 111 and an operation unit 112 are arranged on the surface of apparatus body 110, and the cuff 130 is attached to the apparatus body 110.

Fig. 2 is a cross sectional view showing the interior structure of the blood pressure monitor cuff shown in Fig. 1. As shown in Fig. 2, blood pressure monitor cuff 130 in this embodiment essentially includes a bag-like cover body 140 formed of a cloth or the like, an air bag 150 arranged inside the cover body 140, and a curled elastic member 160 having bending elasticity, which is arranged in the interior of cover body 140 and positioned outside air bag 150 in a fitted state for temporarily fitting the cuff on the wrist. These cover body 140, air bag 150 and curled elastic member 160 extend longitudinally with respect to the direction in which cuff 130 is wrapped (the circumferential direction).

Cover body 140 includes an inner cover 142 positioned inside in a fitted state and formed of a highly stretchy cloth or the like, and an outer cover 141 positioned outside of inner cover 142 and formed of a less stretchy cloth or the like. These inner cover 142 and outer cover 141 are superposed and stitched together at their peripheries thereby to form a bag. A hook-and-loop fastener 155 is provided on the inner circumferential surface side of cover body 140 at one end thereof in the longitudinal direction. A hook-and-loop fastener 156 engaging the hook-and-loop fastener 155 is affixed on the outer circumferential surface of cover body 140 on the other end thereof in the longitudinal direction. These hook-and-loop fasteners 155, 156 are means for stably fixing blood pressure monitor 100 on the wrist.

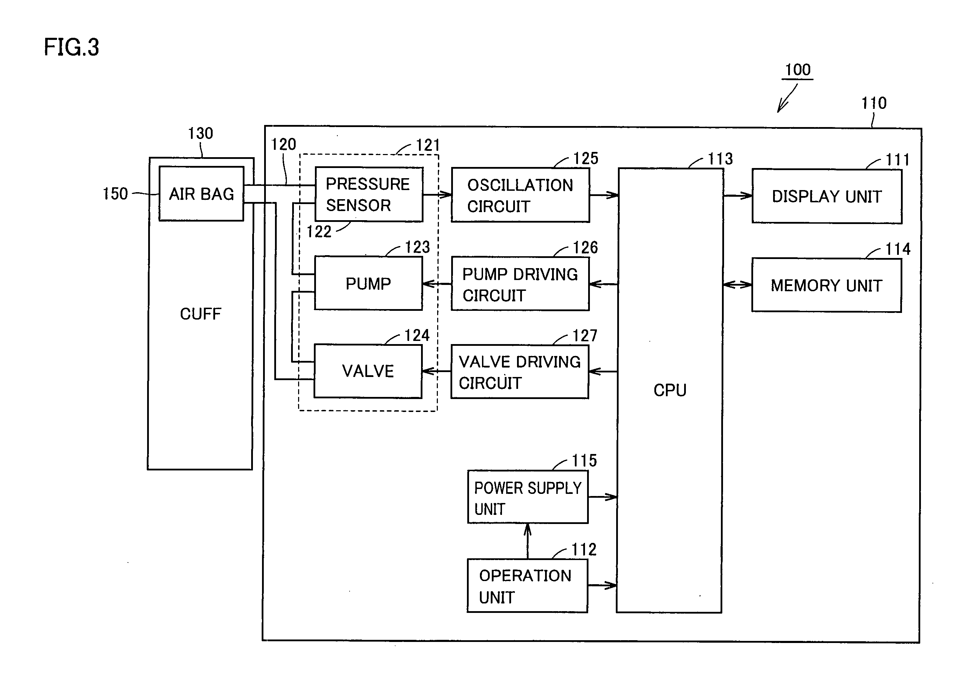

Air bag 150 is formed of a bag-like member formed of a resin sheet and includes an inflation/deflation space therein. The inner circumferential surface of air bag 150 functions as a squeezing surface for squeezing the wrist. The inflation/deflation space is connected to an air system 121 for blood pressure measurement of apparatus body 110 described later through a tube 120 (see Fig. 3).

Curled elastic member 160 is arranged outside air bag 150. Curled elastic member 160 is formed to be elastically deformable in the radial direction when being annually wound around. Curled elastic member 160 is adhered to the outer surface of air bag 150 by not-shown adhesion means such as a double-sided tape. The curled elastic member 160 is formed in such a manner as to keep its annular shape and acts such that air bag 150 tightly fits on a living body in a fitted state. The curled elastic member 160 is formed, for example, of a resin member such as polypropylene so as to develop sufficient elasticity. The detailed structure of curled elastic member 160 will be described later.

Fig. 3 is a block diagram showing the configuration of the blood pressure monitor in this embodiment. As shown in Fig. 3, apparatus body 110 includes an air system 121 for blood pressure measurement for supplying or exhausting air to/from the air bag 150 through a tube 120, and an oscillation circuit 125, a pump driving circuit 126 and a valve driving circuit 127 provided in connection with air system 121 for blood pressure measurement. These components function as an inflation/deflation unit for inflating and deflating air bag 150.

Apparatus body 110 also includes a CPU (Central Processing Unit) 113 for intensively controlling and watching each unit, a memory unit 114 for storing a program causing CPU 113 to execute prescribed operations and a variety of information such as a measured blood pressure value, a display unit 111 for displaying a variety of information including a blood pressure measurement result, an operation unit 112 operated to input a variety of instructions for measurement, and a power supply unit 115 for supplying power to CPU 113 in response to a power-on instruction from operation unit 112. CPU 113 functions as blood pressure value calculation means for calculating a blood pressure value.

Air system 121 for blood pressure measurement includes a pressure sensor 122 for measuring a pressure in air bag 150 (referred to as "cuff pressure" hereinafter), a pump 123 for supplying air to air bag 150, and a valve 124 opened and closed for exhausting or introducing air for air bag 150. Pressure sensor 122 functions as pressure detection means for detecting the cuff pressure. Oscillation circuit 125 outputs to CPU 113 a signal of an oscillation frequency according to an output value from pressure sensor 122. Pump driving circuit 126 controls driving of pump 123 based on a control signal applied from CPU 113. Valve driving circuit 127 controls opening and closing of valve 124 based on a control signal applied from CPU 113.

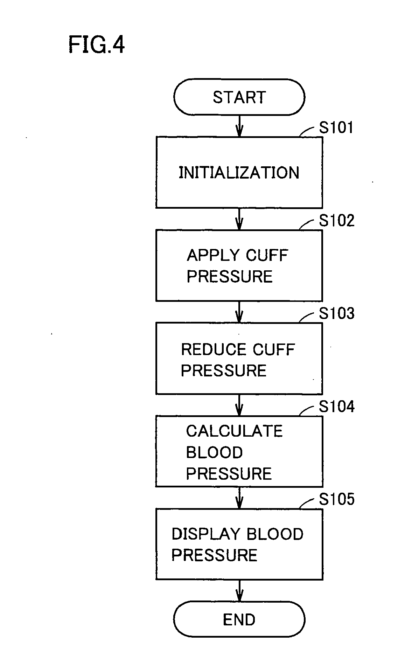

Fig. 4 is a flowchart showing a flow of a blood pressure measurement process in the blood pressure monitor in this embodiment. The program according to this flowchart is stored beforehand in memory unit 114. CPU 113 reads and executes the program from memory unit 114 to perform a blood pressure measurement process.

As shown in Fig. 4, when a subject operates an operation button of operation unit 112 of blood pressure monitor 100 for power-on, blood pressure monitor 100 is initialized (step S101). Then, upon reaching a measurable state, CPU 113 starts driving pump 123 and gradually increases the cuff pressure of air bag 150 (step S102). In the process of gradually applying pressure, when the cuff pressure reaches a prescribed level for blood pressure measurement, CPU 113 stops pump 123 and then gradually opens the originally closed valve to gradually exhaust air from air bag 150 and gradually decrease the cuff pressure (step S103). In this embodiment, a blood pressure is measured in the process of slowly decreasing the cuff pressure.

Then, CPU 113 calculates a blood pressure (a systolic blood pressure value and a diastolic blood pressure value) in a well-known procedure (step S 104). Specifically, in the process of gradually decreasing the cuff pressure, CPU 113 extracts pulse wave information based on an oscillation frequency obtained from oscillation circuit 125. Then, a blood pressure value is calculated based on the extracted pulse wave information. When a blood pressure value is calculated at step S104, the calculated blood pressure value is displayed on display unit 111 (step S105). It is noted that the measurement scheme as described above is based on a so-called depressurization measurement scheme in which a pulse wave is detected during depressurization of the air bag. Alternatively, a so-called pressurization measurement scheme may also be employed, as a matter of course, in which a pulse wave is detected during pressurization of the air bag.

Blood pressure monitor 100 and blood pressure monitor cuff 130 in this embodiment are characterized by the structure of curled elastic member 160 arranged in blood pressure monitor cuff 130. In the following, the structure of curled elastic member 160 will be described in detail with reference to the figures.

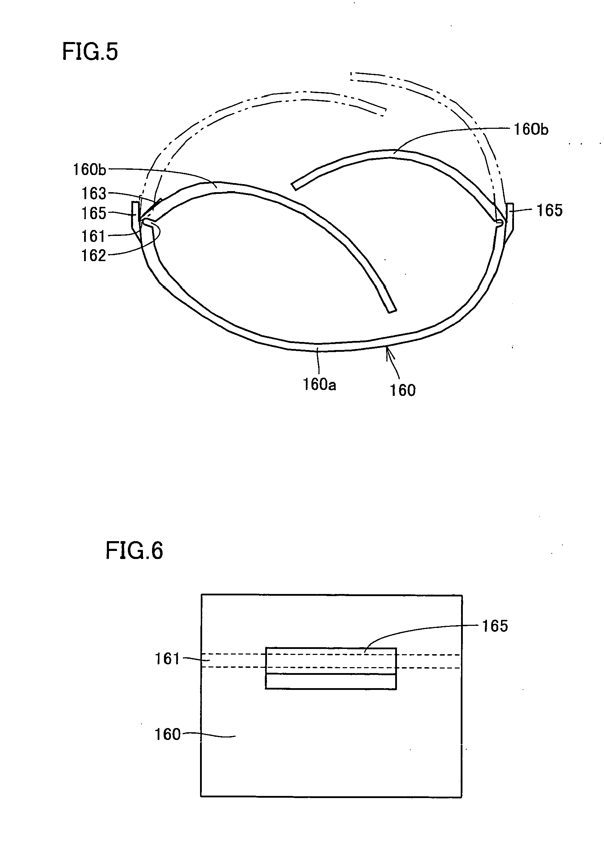

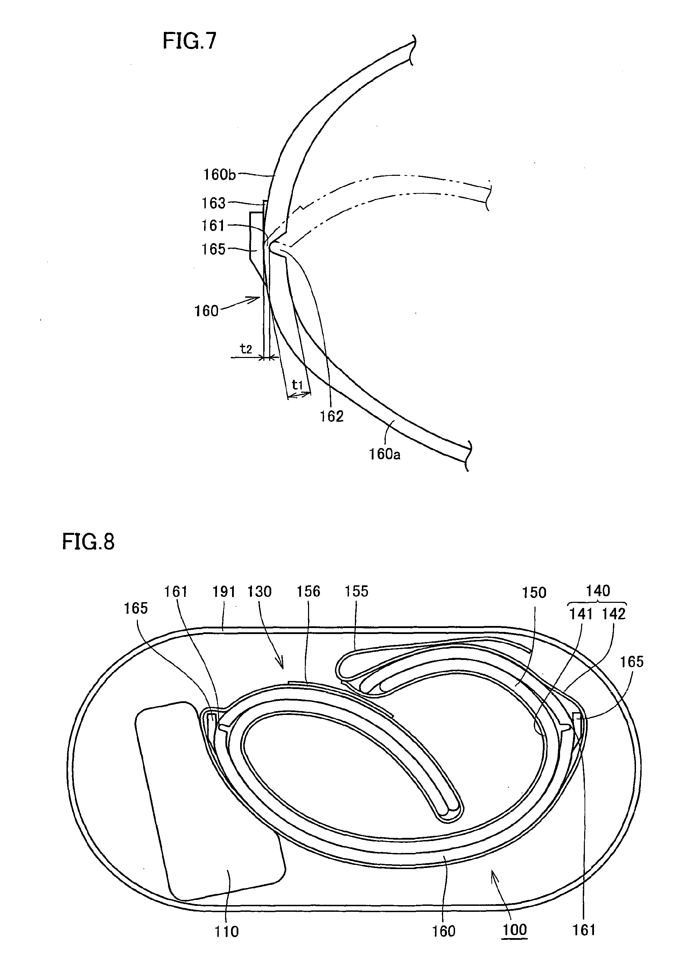

Fig. 5 is a front view and Fig. 6 is a side view showing the structure of the curled elastic member in the present embodiment. Fig. 7 is a front view showing the structure of a flexion portion. The curled elastic member 160 in the present embodiment is formed to have a curved arc-shaped cross section and a length that allows the opposing ends to overlap with each other.

Curled elastic member 160 is provided with a flexion portion 161 that allows for local flexion. In this embodiment, flexion portions 161 are provided at opposing two locations of curled elastic member 160. More specifically, flexion portions 161 are provided at locations each at 90° from the middle portion of the entire length of curled elastic member 160. Curled elastic member 160 is shaped approximately like an ellipse in cross section to be adapted to the shape of the human wrist, and flexion portions 161 are each positioned in the vicinity of the intersections between the ellipse and the longitudinal axis thereof.

In the present embodiment, flexion portions 161 are provided at two locations by way of illustration. However, flexion portion 161 may be provided at only one location. Alternatively, flexion portions 161 may be provided at three or more locations.

A groove 162 is provided on the inner circumferential surface side of flexion portion 161. The groove 162 extends in the direction orthogonal to the circumferential direction of curled elastic member 160 along the entire length of the width of curled elastic member 160. The groove 162 has a groove bottom formed of a curved surface. Specifically, it is arc-shaped in cross section. The curved surface of the groove bottom is not limited to an arc and may be formed of any other quadric curve. Opposing side walls of groove 162 form an approximate V shape when curled elastic member 160 is opened (a state suitable for blood pressure measurement), and they form an approximate U shape when curled elastic member 160 is folded (a state suitable for storage), as shown in Fig. 7.

Referring to Fig. 7, a specific size of curled elastic member 160 is shown by way of example. In this embodiment, thickness $t_1$ of curled elastic member 160 in proximity to flexion portion 161 is 1.0 mm or more and 1.5 mm or less, thickness $t_2$ of the bottom portion of groove 162 is 0.3 mm or more and 0.6 mm or less, and the radius of the arc forming the bottom portion of groove 162 is 0.5 mm or more and 2.0 mm or less.

Here, when curled elastic member 160 is folded, bending moment M (kg·mm) is expressed by the following equation where bending stress is $\sigma_b$ (kg/mm$^2$) and section modulus is Z (mm$^3$).

$$M = \sigma_b \times Z$$

Then, section modulus Z is expressed by the following equation where the width of curled elastic member 160 is W (mm) and the thickness is t (mm).

$$Z = 1/6 \times W \times t^2$$

If the thickness of flexion portion 161 of curled elastic member 160 (the thickness of the groove bottom) is one fifth of the thickness of curled elastic member 160 in proximity to flexion portion 161, the bending moment obtained when flexion portion 161 of curled elastic member 160 is folded is one twenty-fifth of that of a portion other than flexion portion 161 being bent. Curled elastic member 160 is provided with flexion portion 161 having such a groove 162, so that curled elastic member 160 can easily be folded. In addition, the flexion position of curled elastic member 160 can be specified at flexion portion 161 when curled elastic member 160 is folded.

Furthermore, groove 162 is provided on the inner circumferential surface side of curled elastic member 160 with its groove bottom having the shape of an arc in cross section, so that compression stress is applied in a distributed manner over the entire groove bottom of groove 162 when curled elastic member 160 is folded. Therefore, stress concentration at flexion portion 161 at the time of folding can be reduced.

This effect is more evident as compared with the case where a groove is provided on the outer surface of curled elastic member 160 in contrast to the present embodiment. When a groove is provided on the outer surface of curled elastic member 160, a linear portion on the inner circumferential surface of curled elastic member 160 that corresponds to the groove is locally bent, and compression stress concentrates on this portion. In this case, the linear portion is easily broken. By contrast, in the present embodiment, groove 162 is provided on the inner surface of curled elastic member 160 and the groove bottom is arc-shaped in cross section, so that the bottom portion of groove 162 of flexion portion 161 keeps the shape of a curved-surface in cross section even when curled elastic member 160 is folded. Therefore, compression stress can be distributed over the entire groove bottom, thereby avoiding stress concentration. As a result, the strength of curled elastic member 160 is enhanced.

An abutment piece 165 in abutment with the outer surface of curled elastic member 160 is provided on the outer circumferential side of curled elastic member 160 at flexion portion 161. Abutment piece 165 is a rectangular plate-like body integrally formed with curled elastic member 160. As shown in Fig. 6, abutment piece 165 is provided at the middle portion in the width direction of curled elastic member 160 and has a length about a half the entire width of curled elastic member 160.

Abutment piece 165 is coupled to the middle portion 160a side from flexion portion 161, in proximity to flexion portion 161. The upper end of abutment piece 165 as viewed in Fig. 5 and Fig. 6 is a free end. The inner surface of abutment piece 165 abuts on the end portion 160b side from flexion portion 161 (an abutment portion 163), in proximity to flexion portion 161. Abutment portion 163 in abutment with abutment piece 165 is planar and is brought into intimate contact with the inner surface of abutment piece 165 when curled elastic member 160 is opened. In contrast to the present embodiment, abutment piece 165 may be coupled to the end portion 160 side and may have its inner surface in abutment with the middle portion 160a side.

In curled elastic member 160 of the present embodiment, flexion portions 161 are provided at opposing two locations, so that curled elastic member 160 can be folded inwardly at the end portion side 160b extending from flexion portion 161 of curled elastic member 160.

Fig. 8 is a cross sectional view showing that the curled elastic member is folded. Curled elastic member 160 can be folded inwardly, so that cuff 130 containing curled elastic member 160 therein can also be folded into a compact shape. Accordingly, blood pressure monitor 100 can be stored in a slim storage case 191 as shown in Fig. 8, resulting in a readily-portable blood pressure monitor .

In the present embodiment, abutment piece 165 is provided on the outer side of flexion portion 161 of curled elastic member 160, and abutment piece 165 prevents curled elastic member 160 from opening further than a prescribed angle. In the conventional curled elastic member with a groove provided on the outer side of the curled elastic member and having its inside surfaces mutually abutting against each other, the thickness of the curled elastic member at the flexion portion needs to be made large enough. However, curled elastic member 160 of the present embodiment is not required to do so. Therefore, curled elastic member 160 in the present embodiment may employ an optimum thickness for obtaining the functional characteristics essentially required of curled elastic member 160.

(Second Embodiment)

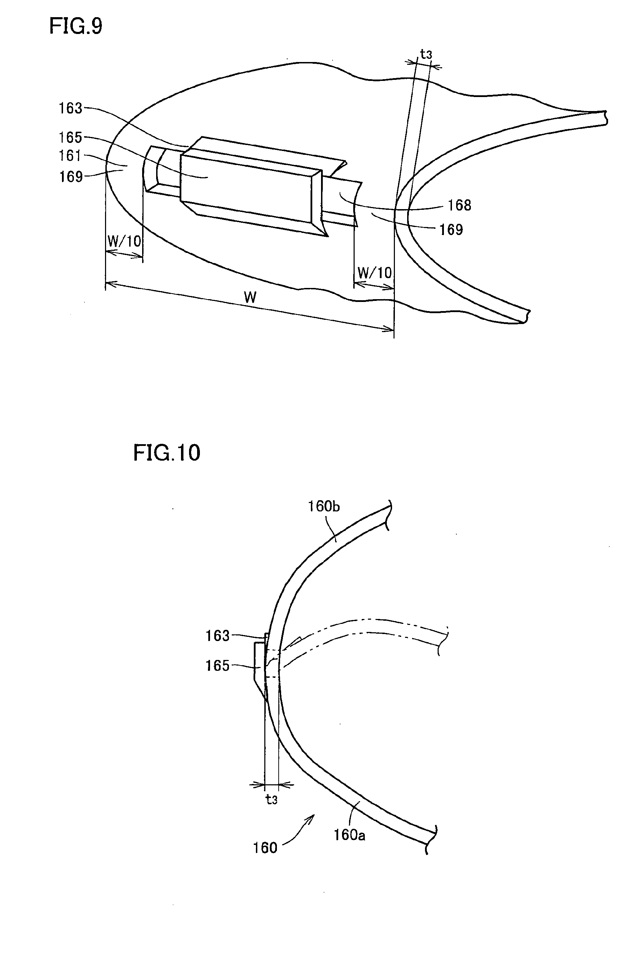

A second embodiment will now be described with reference to the figures. Fig. 9 is a perspective view and Fig. 10 is a front view showing the structure of the flexion portion of the curled elastic member.

In curled elastic member 160 of the second embodiment, flexion portion 161 is provided with a rectangular opening portion 168 extending in the width direction of curled elastic member 160. The area per unit length in the circumferential direction of curled elastic member 160 at flexion portion 161 is formed to be smaller than that of any other portion of curled elastic member 160. More specifically, when the entire width of curled elastic member 160 is represented by W, opening portion 168 is provided to extend over the length of eight tenths of W at the middle portion while connection portions 169 each having a width of one tenth of W are left at opposing ends in the width direction of flexion portion 161.

Based on the equation above, when flexion portion 161 provided with opening portion 168 is folded, the bending moment of curled elastic member 160 in the present embodiment is one fifth of that without opening portion 168. Accordingly, curled elastic member 160 can be folded easily at flexion portion 161, and in addition, the flexion position of curled elastic member 160 can be specified at flexion portion 161. In the present embodiment, thickness $t_3$ of connection portion 169 of flexion portion 161 is 0.8 mm or more and 1.2 mm or less. The width or thickness of connection portion 169 can vary according to the material of curled elastic member 160 and the like.

Abutment piece 165 is provided on the outer side of flexion portion 161, similarly to the first embodiment. The abutment piece 165 is coupled to one edge portion (the lower side in Fig. 9 and Fig. 10) of opening portion 168 and abuts against abutment portion 163 in the vicinity of the other edge portion (the upper side in Fig. 9 and Fig. 10) of opening portion 168. This can prevent curled elastic member 160 from opening further than a prescribed angle when curled elastic member 160 is opened.

In the present embodiment, with provision of opening portion 168 extending in the width direction, the area per unit length in the circumferential direction of curled elastic member 160 at flexion portion 161 is smaller than that of any other portion of curled elastic member 160. Alternatively, cuts may be provided at opposing sides of curled elastic member 160 in place of opening portion 168 at flexion portion 161. The opening portion and the cuts may both be provided.

In the present embodiment, in place of groove 162 of the first embodiment, opening portion 168 is provided. However, groove 162 and opening portion 168 may both be provided. In other words, grooves 162 may be provided at connection portions 169 on the opposing sides of opening portion 168.

In these two embodiments as described above, curled elastic member 160 is opened (a state suitable for blood pressure measurement) in an initial state (natural state) and is folded by flexing flexion portion 161 during storage, by way of illustration. On the contrary, curled elastic member 160 may be formed such that curled elastic member 160 is folded by flexing flexion portion 161 in an initial state, and curled elastic member 160 may be opened by extending flexion portion 161 when fitted on a human body.

Although the present invention has been described and illustrated in detail, it is clearly understood that the same is by way of illustration and example only and is not to be taken by way of limitation, the spirit and scope of the present invention being limited only by the terms of the appended claims.

**Claims**

1. A blood pressure monitor cuff (130) comprising a curled elastic member (160) having a flexion portion (161) allowing for local flexion, wherein
said curled elastic member (160) is provided with an abutment piece (165) positioned on an outer side of said flexion portion (161) and brought into abutment with an outer surface in proximity to said flexion portion (161) when said curled elastic member (160) is in the shape suitable for blood pressure measurement.

2. The blood pressure monitor cuff according to claim 1, wherein said flexion portion (161) has a groove (162) provided on an inner circumferential side thereof and extending in a direction orthogonal to a circumferential direction of said curled elastic member (160).

3. The blood pressure monitor cuff according to claim 2, wherein a groove bottom of said groove (162) is formed of a curved surface.

4. The blood pressure monitor cuff according to claim 1, wherein said flexion portion (161) is formed such that an area per unit length in a circumferential direction of said curled elastic member (160) is smaller than that of any other portion of said curled elastic member (160).

5. A blood pressure monitor comprising:

    the blood pressure monitor cuff (130) according to claim 1;
    a fluid bag (150) inflated and deflated as a fluid comes in and out;
    an inflation/deflation unit (123, 124) for inflating and deflating said fluid bag (150);
    a pressure detection unit (122) detecting a pressure in said fluid bag (150); and
    a blood pressure value calculation unit (113) calculating a blood pressure value based on information of the pressure detected by said pressure detection unit (122).

FIG.1

111

100

112

110

130

FIG.2

156

130

155

150

141
142 } 140

110

160

100

**FIG.3**

EP 1 702 562 A1

# FIG.4

```
        ( START )
            │
            ▼  ⟋ S101
    ┌───────────────────┐
    │  INITIALIZATION   │
    └───────────────────┘
            │
            ▼  ⟋ S102
    ┌───────────────────┐
    │   APPLY CUFF      │
    │   PRESSURE        │
    └───────────────────┘
            │
            ▼  ⟋ S103
    ┌───────────────────┐
    │   REDUCE CUFF     │
    │   PRESSURE        │
    └───────────────────┘
            │
            ▼  ⟋ S104
    ┌───────────────────┐
    │   CALCULATE       │
    │   BLOOD           │
    │   PRESSURE        │
    └───────────────────┘
            │
            ▼  ⟋ S105
    ┌───────────────────┐
    │  DISPLAY BLOOD    │
    │  PRESSURE         │
    └───────────────────┘
            │
            ▼
         ( END )
```

FIG.5

160b

160b
163
165
161
162

165

160a    160

FIG.6

165

161

160

FIG.7

FIG.8

## FIG.9

## FIG.10

**European Patent Office**

**EUROPEAN SEARCH REPORT**

Application Number

EP 06 00 5217

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PATENT ABSTRACTS OF JAPAN vol. 1996, no. 05, 31 May 1996 (1996-05-31) & JP 08 010233 A (SATO IKEN:KK), 16 January 1996 (1996-01-16) * abstract * | 1-5 | INV. A61B5/022 |
| A | US 2003/220575 A1 (FUMURO SHINICHI ET AL) 27 November 2003 (2003-11-27) * paragraph [0007] - paragraph [0022] * * paragraph [0064] * * figures 1a,12a,12b * | 1,5 | |
| A | PATENT ABSTRACTS OF JAPAN vol. 018, no. 381 (C-1226), 18 July 1994 (1994-07-18) & JP 06 105813 A (MATSUSHITA ELECTRIC WORKS LTD), 19 April 1994 (1994-04-19) * abstract * | 1,5 | |

TECHNICAL FIELDS SEARCHED (IPC)

A61B

The present search report has been drawn up for all claims

| Place of search | Date of completion of the search | Examiner |
|---|---|---|
| Berlin | 7 July 2006 | Abraham, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 06 00 5217

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2006

| Patent document cited in search report | | Publication date | Patent family member(s) | | Publication date |
|---|---|---|---|---|---|
| JP 08010233 | A | 16-01-1996 | JP | 2796938 B2 | 10-09-1998 |
| US 2003220575 | A1 | 27-11-2003 | CN | 1449719 A | 22-10-2003 |
| | | | EP | 1352612 A2 | 15-10-2003 |
| | | | JP | 2003299626 A | 21-10-2003 |
| JP 06105813 | A | 19-04-1994 | JP | 3151307 B2 | 03-04-2001 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6105813 A